# EUROPEAN PATENT SPECIFICATION

(11) **EP 2 661 274 B1**
(45) Date of publication and mention of the grant of the patent: **22.05.2019**
(21) Application number: 11854913.8
(22) Date of filing: 06.01.2011
(51) Int. Cl.: A61K 36/886, A61K 31/715, A61K 9/14, A61P 35/00, A61P 37/02, A61P 37/04, C08B 37/04, C08L 5/00, A61K 9/00, A61K 45/06, A61K 9/08, A61K 9/19, A61K 31/736

(54) **COMPOSITIONS AND METHODS OF ALOE POLYSACCHARIDES**
ZUSAMMENSETZUNGEN UND VERFAHREN FÜR ALOEPOLYSACCHARIDE
COMPOSITIONS ET PROCÉDÉS LIÉS À DES POLYSACCHARIDES D'ALOE

(43) Date of publication of application: 13.11.2013
(73) Proprietor: Bespoke Bioscience, LLC, Dallas, TX 75254 (US)
(72) Inventor: DANHOF, Ivan, E., Grand Prairie, TX 75050 (US)
(74) Representative: Sonn & Partner Patentanwälte
(86) International application number: PCT/US2011/020402
(87) International publication number: WO 2012/094010

(56) References cited:
- WO-A1-90/01253
- WO-A2-00/41541
- US-A- 5 106 616
- US-A- 5 118 673
- US-A- 5 703 060
- US-A- 5 773 425
- US-A1- 2003 096 378
- US-A1- 2006 084 629
- US-B2- 7 196 072
- IM S A ET AL: "Identification of optimal molecular size of modified Aloe polysaccharides with maximum immunomodulatory activity", INTERNATIONAL IMMUNOPHARMACOLOGY, vol. 5, no. 2, 1 February 2005 (2005-02-01), pages 271-279, XP027686394, ELSEVIER, AMSTERDAM, NL ISSN: 1567-5769 [retrieved on 2005-02-01]
- PI W -X ET AL: "Purification process of aloe polysaccharide and its anti-tumor activity in vitro", CHINESE JOURNAL OF NATURAL MEDICINES, vol. 5, no. 6, November 2007 (2007-11), pages 425-427, XP009177942, CN ISSN: 1672-3651
- QIU Z ET AL: "Modified Aloe barbadensis polysaccharide with immunoregulatory activity", PLANTA MEDICA, vol. 66, no. 2, 1 March 2000 (2000-03-01), pages 152-156, XP009177943, THIEME VERLAG, DE ISSN: 0032-0943
- DATABASE TCM SIPO; 18 May 2007 (2007-05-18), Li Fenfang et al.: "Preparation method of Aloe polysaccharide powder and Aloe activated water from Aloe", XP002724294, Database accession no. CN-200710034943-A & CN 101 070 354 A (UNIV ZHONGNAN [CN] UNIV CENTRAL SOUTH) 14 November 2007 (2007-11-14)
- FACHET J ET AL: "Modulation of immune responses and defense against tumor by a polymannan- -polyglycan polysaccharide: mannosym", INTERNATIONAL JOURNAL OF IMMUNOPHARMACOLOGY, vol. 2, no. 3, 1 January 1980 (1980-01-01) , page 185, XP023812197, ELMSFORD,NY, US ISSN: 0192-0561, DOI: 10.1016/0192-0561(80)90105-8 [retrieved on 1980-01-01]
- LEUNG M Y K ET AL: "Polysaccharide biological response modifiers", IMMUNOLOGY LETTERS, vol. 105, no. 2, 15 June 2006 (2006-06-15) , pages 101-114, XP024999179, ELSEVIER BV, NL ISSN: 0165-2478, DOI: 10.1016/J.IMLET.2006.01.009 [retrieved on 2006-06-15]
- DATABASE WPI Week 200920 Thomson Scientific, London, GB; AN 2009-B48662 XP002724303, & CN 101 306 012 A (UNIV NO 4 MILITARY MEDICINE PLA) 19 November 2008 (2008-11-19)
- DATABASE TCM [Online] SIPO; 4 February 2001 (2001-02-04), Yang Mengjun: "Nano Angelicae longhui medicine and its preparation", XP002724304, Database accession no. CN-01100154-A & CN 1 362 105 A (YANG MENGJUN [CN]) 7 August 2002 (2002-08-07)
- ANDERSEN F A: "Final report on the safety assessment of aloe andongensis extract, aloe andongensis leaf juice, aloe arborescens leaf extract, aloe arborescens leaf juice, aloe arborescens leaf protoplasts, aloe barbadensis flower extract, aloe barbadensis leaf, aloe barbadensis leaf extract, aloe barbadensis leaf", INTERNATIONAL JOURNAL OF TOXICOLOGY, vol. 26, no. SUPPL. 2, 2007, pages 1-50, XP009177941, GB ISSN: 1091-5818
- 'Acemannan Immunostimulant' DRUGS.COM, [Online] 31 December 2008, XP003032749 Retrieved from the Internet: <URL:http://www.drugs.com/vet/acemannan-imm unostimulant.html> [retrieved on 2011-03-22]
- EGGER, S.F. ET AL.: 'Hematopoietic augmentation by a beta-(1,4)-linked mannan' CANCER IMMUNOLOGY, IMMUNOTHERAPY. vol. 43, no. 4, 1996, pages 195 - 205, XP009177929
- PENG S.Y. ET AL.: 'Decreased mortality of Norman Murine Sarcoma in mice treated with the immunomodulator, Acemannan' MOLECULAR BIOTHERAPY vol. 3, 1991, pages 79 - 87, XP009142243

## Description

### TECHNICAL FIELD OF THE INVENTION

The present invention relates in general to the field of aloe polysaccharides, and more particularly, to compositions of aloe polysaccharides and use of such compositions for as immunomodulators and for the treatment of different types of cancers.

### BACKGROUND ART

Without limiting the scope of the invention, its background is described in connection with compositions, methods of preparation, and therapeutic uses of Aloe Vera polysaccharides.

United States Patent No. 7,196,072 issued to Pasco et al. (2007) describes a complex, water soluble polysaccharide fraction having potent immunostimulatory activity isolated from *Aloe vera.* The polysaccharide fraction has an apparent molecular weight above 2 million daltons with glucose, galactose, mannose and arabinose as its major components. The invention further describes pharmaceutical compositions containing the instant polysaccharide fraction, optionally in combination with acceptable pharmaceutical carriers and/or excipents. These pharmaceutical compositions may be used to provide immunostimulation to an individual in need of such treatment by administering to such an individual an effective amount of the composition.

United States Patent No. 6,083,508 issued to Avalos and Danhof (2000) describes a process for forming an aloe product from only the leaf residue obtained after filleting aloe leaves having an internal fillet which is removed therefrom. According to the '508 patent the residue is formed into a slurry by grinding and the aloe product is generated from the slurry. In addition the steps of preparing the aloe product comprises cleansing an aloe leaf before filleting it, separating the slurry formed into a liquid and solids, and further treating the separated liquid to remove laxatives before forming the aloe product. Also, a process including all of the above steps may also be performed in order to form the liquid.

United States Patent Application No. 2006/0084629 (Needleman and Needleman, 2006) discloses a combination of two, specialized, high molecular weight-long chain fractions isolated from Aloe Vera and Maitake TD to stimulate immune system activity comprising long chain-high molecular weight polysaccharides which activate the body's natural immune response, triggering an increase in the production of macrophages, T-cells, B-cells, natural killer cells, cytokines and antibodies. These long chain polysaccharides together with other active ingredients may provide proper immune system support thereby preventing debilitating diseases such as cancer, heart disease and aging.

United States Patent No. 5,773,425 issued to Carrington Laboratories, Inc. describes a process for forming an aloe product using a stabilised, liquid Aloe vera as starting material, which is precipitated with primarily ethanol and then freeze-dried.

### DISCLOSURE OF THE INVENTION

The present invention describes an aloe polysaccharide composition and the use of the composition as an immunomodulating agent and for the treatment of different types of cancers selected from leukemias and lymphomas, prostate cancer, breast cancer, and colon cancer, immune diseases, particularly immune related neoplasms.

The present invention provides a method for preparing a fine powder of a polymannan extract as end product comprising the steps of: (i) weighing a specified quantity of a freeze-dried aloe powder as source product, wherein the quantity is corrected for a moisture content, (ii) dissolving the freeze-dried aloe powder in deionized water to form a solution, (iii) adding a first volume of an alcoholic solvent to the solution to form a first mixture; wherein the alcoholic solvent to the deionized water ratio is at least 2.5:1, (iv) allowing the first mixture to settle for at least 8 hours, (v) withdrawing a volume of a supernatant solution from the first mixture and adding a second volume of the alcoholic solvent to the volume of the supernatant solution to form a second mixture, (vi) centrifuging the second mixture, (vii) observing for a presence of a precipitate in the second mixture, (vii) adding a third volume of the alcoholic solvent to the first mixture if any precipitate is observed in the second mixture, (viii) decanting the supernatant of the first mixture by siphoning, wherein the decantation is done only if no precipitate is observed in the second mixture, (ix) filtering the precipitate from the first mixture by using a filter paper and a suction funnel under vacuum, (x) recovering the powder of the polymannan extract from the suction funnel by scraping, (xi) placing the powder of the polymannan extract in a capped lyophilization flask in a freezer for at least 8 hours, (xii) lyophilizing the frozen powder of the polymannan extract in a lyophilizer, and (xiii) grinding the lyophilized powder of the polymannan extract in a grinder to a very fine texture.

In one aspect the method further comprises the step of weighing, labeling, and storing the fine powder of the polymannan extract in a container. The freeze-dried aloe powder source product as described in the embodiment of the present invention is derived from an Aloe species selected from the group consisting of *Aloe vera, Aloe arborescens, Aloe aristata, Aloe dichotoma, Aloe nyeriensis, Aloe variegate, Aloe barbadensis, and Aloe wildii.* The freeze-dried aloe powder source product used in the present invention comprises aloe polysaccharides, wherein the aloe polysaccharides comprise one or more small chain, medium chain, large chain, very-large chain polysaccharides, or any combinations thereof. In a specific aspect the alcoholic solvent is selected from the group consiting of methanol, ethanol, isopropyl alcohol, and propanol. In another aspect the aloe polysaccharides further comprise simple sugars, selected from the group consisting of glucose, mannose, arabinose, and galactose and have a molecular weight ranging from 11,500 Daltons to over 10,000,000 Daltons.

In another aspect the freeze-dried aloe powder has at least 25% of aloe polysaccharides. In yet another aspect the freeze-dried aloe powder has 25%, 30%, 40%, 50%, 60%, 70%, 80%, 90%, and 95% aloe polysaccharides. The freeze dried aloe powder as described in the method of the present invention comprises at least 14% of aloe polysaccharides having a molecular weight of 66,000 Daltons, at least 9% of aloe polysaccharides having a molecular weight of 480,000 Daltons, at least 3.5% of aloe polysaccharides having a molecular weight of 1,000,000 Daltons, at least 2.4% of aloe polysaccharides having a molecular weight of 2,000,000 Daltons.

In a specific aspect of the method relating to the aloe polysaccharide composition in the freeze dried aloe powder about 1.32%-6.36% of the aloe polysaccharides have a molecular weight of 2,000,000 Daltons, 2.55%-3.89% have a molecular weight of 1,000,000, and 63.85%-73.36%. of aloe polysaccharides have a molecular weight of 480,000 Daltons. In one aspect the freeze dried aloe powder may contain one or more residual small molecular weight species, selected from the group consisting of glucose, organic acids, lactic acid, malic acids, citric acids, and aspartic acid. In another aspect the one or more residual small molecular weight species are present in amounts ranging from 14%-24%. In yet another aspect the fine powder of the polymannan extract is used on the preparation of a pharmaceutical composition to be used in the treatment of one or more malignacies selected from the group consisting of leukemias and lymphomas, prostate cancer, breast cancer, and colon cancer, and for the treatment of one or more immune disorders.

In another embodiment the present invention discloses a sterile injectable formulation of a polymannan extract comprising: a specified quantity of very fine polymannan extract dissolved in deionized water and one or more pharmaceutical preservatives. The one or more pharmaceutical preservatives the may be used in the formulation described hereinabove is selected from the group consisting of parabens, benzoic acid and their salts, mercurials, quarternary ammonium salts, benzyl alcohol and other related alcohols, and phenols. In a specific aspect the preservative is benzyl alcohol. In one aspect the polymannan extract comprises aloe polysaccharides, wherein the aloe polysaccharides comprise one or more small chain, medium chain, large chain, very-large chain polysaccharides, or any combinations thereof. In another aspect the aloe polysaccharides further comprise simple sugars, selected from the group consisting of glucose, mannose, arabinose, and galactose.

In other specific aspects the aloe polysaccharides have a molecular weight ranging from 11,500 Daltons to over 10,000,000 Daltons and the polymannan extract has at least 25% of aloe polysaccharides, wherein the polymannan extract has 25%, 30%, 40%, 50%, 60%, 70%, 80%, 90%, and 95% aloe polysaccharides. In a related aspect the polymannan extract comprises at least 14% of aloe polysaccharides having a molecular weight of 66,000 Daltons, at least 9% have a molecular weight of 480,000 Daltons, at least 3.5% have a molecular weight of 1,000,000 Daltons, and at least 2.4% of aloe polysaccharides having a molecular weight of 2,000,000 Daltons. The composition of the present invention is used in the treatment of one or more cancers selected from the group consisting of leukemias and lymphomas, prostate cancer, breast cancer, and colon cancer, for immunomodulation, immunostimulation, or for the treatment of an individual with a compromised immune system or an immune disease. The composition of the present invention causes a 75-80% increase in one or more natural killer (NK) cells.

In yet another embodiment the present invention describes a treatment for one or more cancers selected from the group consisting of leukemias and lymphomas, prostate cancer, breast cancer, and colon cancer, comprising the steps of: identifying an individual in need of treatment against the one or more cancers and injecting a sterile injectable polymannan extract formulation two to three times in a week in a dosage sufficient to treat the one or more cancers, wherein the sterile injectable polymannan extract formulation comprises a specified quantity of very fine polymannan extract dissolved in deionized water; and one or more pharmaceutical preservatives. The method further comprises the steps of: withdrawing blood samples from the individual at one or more specified intervals and measuring a level of a caspase 3 protein in the blood and comparing the level obtained with the level prior to the injection, wherein an increased level in caspase 3 is directly related to an increased level of apoptosis of the one or more cancer cells.

In one aspect the dosage of the sterile injectable polymannan extract formulation is dependent on a weight, an age, an ethnicity, and a gender of the individual. In another aspect the polymannan extract comprises aloe polysaccharides, wherein the aloe polysaccharides comprise one or more small chain, medium chain, large chain, very-large chain polysaccharides, or any combinations thereof. In yet another aspect the aloe polysaccharides have a molecular weight ranging from 11,500 Daltons to over 10,000,000 Daltons. The polymannan extract as described in the method of the present invention has at least 25% of aloe polysaccharides. The polymannan extract of the method of the present invention causes a 75-80% increase in one or more natural killer (NK) cells.

In one embodiment the present invention discloses a method of immunomodulation or immunostimulation in an individual with a compromised immune system or an immune disease comprising the steps of: (i) identifying the individual with the compromised immune system or an immune disease and in need of immunomodulation or immunostimulation, (ii) administering intravenously a specified dosage of a sterile injectable polymannan extract formulation, wherein the sterile injectable polymannan extract formulation comprises a specified quantity of very fine polymannan extract dissolved in deionized water; and one or more pharmaceutical preservatives, wherein the dosage of the sterile injectable polymannan extract formulation is dependent on a weight, an age, an ethnicity, and a gender of the individual, (iii) withdrawing blood samples from the individual at one or more specified intervals, and measuring a level of a tumor necrosis factor-alpha (TNFα) in the blood and comparing the level obtained with the level prior to the injection; wherein an increased level in the TNFα indicates immunomodulation or immunostimulation. In a specific aspect the immune disease is an immune related neoplasm. In one aspect the polymannan extract comprises aloe polysaccharides, wherein the aloe polysaccharides comprise one or more small chain, medium chain, large chain, very-large chain polysaccharides, or any combinations thereof. In another aspect the polymannan extract causes a 75-80% increase in one or more natural killer (NK) cells. In yet another aspect the polymannan extract has at least 25% of aloe polysaccharides.

### DESCRIPTION OF THE DRAWINGS

For a more complete understanding of the features and advantages of the present invention, reference is now made to the detailed description of the invention along with the accompanying figures and in which:
**FIG. 1** shows a size-exclusion chromatogram of an aloe polysaccharide showing the retention times of the different glucose and mannose sub-units;
**FIG. 2** shows the size-exclusion chromatogram of an aloe polysaccharide showing the peaks corresponding to the different glucose and mannose sub-units;
**FIG. 3** is the proton-nuclear magnetic resonance profile of the polymannan extract of the present invention;
**FIG. 4A** is a HPLC chromatogram showing the amounts of polysaccharide in each polysaccharide molecular group in a methanol precipitated aloe polysaccharide concentrate;
**FIG. 4B** is the proton-nuclear magnetic resonance profile of a methanol precipitated aloe polysaccharide concentrate;
**FIG. 5A** is a HPLC chromatogram showing the amounts of polysaccharide in each polysaccharide molecular group in a ethanol precipitated aloe polysaccharide concentrate;
**FIG. 5B** is the proton-nuclear magnetic resonance profile of a ethanol precipitated aloe polysaccharide concentrate;
**FIG. 6A** is a HPLC chromatogram showing the amounts of polysaccharide in each polysaccharide molecular group in an isopropyl alcohol precipitated aloe polysaccharide concentrate;
**FIG. 6B** is the proton-nuclear magnetic resonance profile of an isopropyl alcohol precipitated aloe polysaccharide concentrate;
**FIG. 7A** is a HPLC chromatogram showing the amounts of polysaccharide in each polysaccharide molecular group in a propanol precipitated aloe polysaccharide concentrate; and
**FIG. 7B** is the proton-nuclear magnetic resonance profile of a propanol precipitated aloe polysaccharide concentrate.

### Description of the Invention

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The present invention describes a process for preparing a polymannan extract and the use of the said extract in the form of an injection as an immune stimulatory compound. Immune stimulation is assessed using macrophages/monocytes of human origin and the cell type is assessed for the secretion of tumor necrosis factor alpha (TNFα).

Aloe polysaccharides are generally considered to be those molecules composed predominantly of glucose and mannose simple sugars having chain lengths of 10,000 Daltons to those with molecular weights of 10,000,000 Daltons. The higher the mannose content and longer the chain length the greater is the immunomodulatory activity expressed by the polysaccharides. The different long, unbranched chains comprising these aloe polysaccharides are listed in Table 1.

**Table 1: Aloe polysaccharide compositions and pharmacological actions.**

| | # of Sugar Residues | Mol. Wt (Daltons) | Pharmacological Actions |
|---|---|---|---|
| Small Chain Polysaccharides | 70-650 | 11,500- > 100,000 | Diabetes |
| | | | Tyrosinase inhibition (skin lightening) |
| | | | Anti-inflammatory activity (Cox-2 inhibition) |
| Medium Chain Polysaccharides | 1500 | 250,000 | Anti-oxidant |
| | | | Protects heart, lungs (emphysema), and nervous system (Parkinsonism) |
| Large Chain Polysaccharides | 4000-5000 | 650,000 | Antibacterial |
| | | | Induction of healing |
| Very Large Polysaccharides | 8000-9000 | 2,000,000 - 10,000,000 | Immunomodulatory activity |
| | | | Stimulation of β-lymphocytes with the elaboration of antibodies |
| | | | Increasing level of natural killer cells |
| | | | Release of large quantities of TNFα to cause angiogenesis in wounds and promotion of healing |

Aloe polysaccharides with molecular weights of 100,000 Daltons or more are listed in Table 2.

**Table 2: Compositions and molecular weights of aloe polysaccharides having Mol. Wts of 100,000 or greater.**

| | Mol. Wt (Daltons) | Major Components |
|---|---|---|
| Aloeride | 2,000,000-10,000,000 | Arabinose, galactose, glucose, mannose |
| Acemannan | 900,000-1,500,000 | Glucose, mannose |
| Manapol | 500,000-900,000 | Glucose, mannose |
| Aloemannan | 100,000-500,000 | Glucose, Mannose |

The precursor material for the polymannan extract is described by the inventors in an previous patent (US Patent No. 6,083,508- Avalos and Danhof, 2000) titled "*Method of Processing Aloe Leaves*". The certificate of analysis of the polymannan extract precursor material is presented in Table 3.

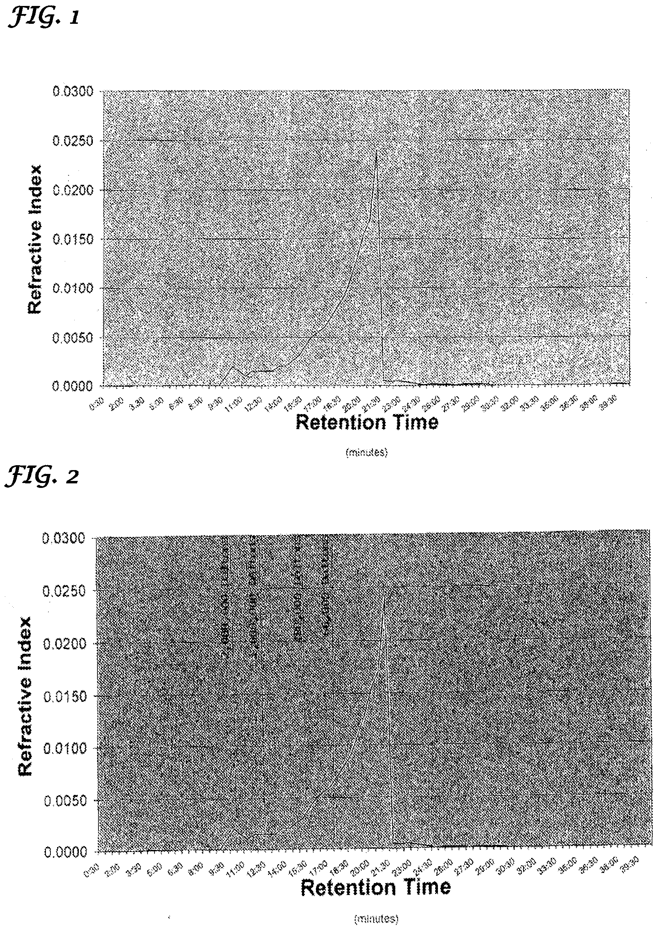

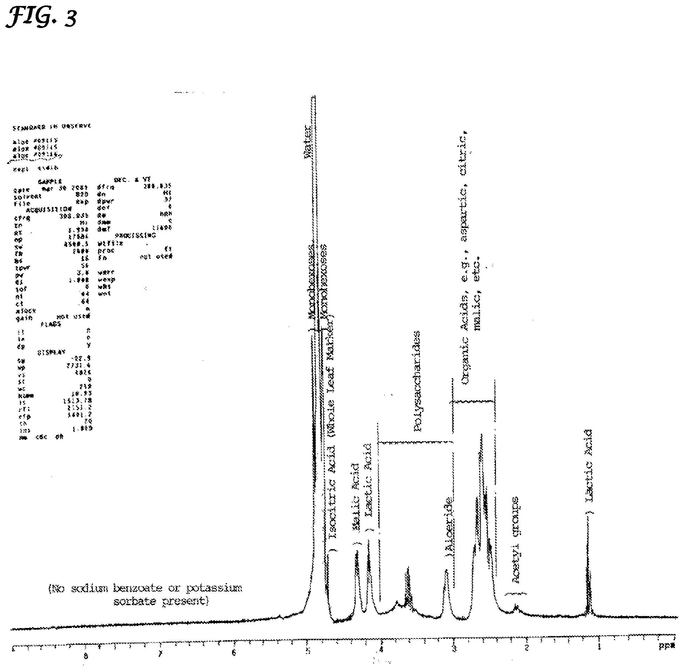

FIG. 1 is a size-exclusion chromatogram of an aloe polysaccharide preparation showing retention times of various-sized glucose and mannose subunits. FIG. 2 is a size-exclusion chromatogram of an aloe polysaccharide preparation identifying molecular weights ranging from 100 to 10,000,000 Daltons. FIG. 3 is the proton-nuclear magnetic resonance profile of the polymannan extract of the present invention. FIG. 3 shows: (i) the absence of the standard preservatives - sodium benzoate and potassium sorbate, (ii) the presence of smaller monohexoses, (iii) the peaks of isocitric acid indicating a whole leaf methodology was employed in processing the raw aloe material, (iv) the presence of malic acid peaks - a primary marker for *Aloe vera,* (v) the presence of the aloeride/acemannan peak in the polysaccharide portion of the profile confirming the presence of the large polysaccharide species, and (vi) acetyl groups are present confirming the presence of the partially acetylated polysaccharide glucomannans.

Size exclusion chromatography o(SEC)f an aloe preparation prior to polymannan extraction: Equipment: HPLC system is a Hitachi L-7100 pump and 7250 autosampler paired to a Waters 410 differential refractometer. The SEC is a Tosoh Biosep G6000 PWXL TSK Gel 30 cm x 7.8 mm operated in a column heater at 70°C. Molecular weight standards are from Sigma - 2,000,000 Daltons, 1,000,000 Daltons, 480,000 Daltons, 66,000 Daltons, and 180 Daltons (glucose). The mobile phase is de-ionized water with flow rate of 0.70 mL/min. The injection volume is 10 uL. The SEC method is described by Pugh et al. (2001).1

**Table 3: Certificate of analysis of Polymannan Extract Precursor Material.**

| **Parameter** | **Constituent Ranrge** | **Determined Value** | **Assessment** |
|---|---|---|---|
| Powder: | | | |
| Color: | White to light tan | Offwhite | Complies |
| Characteristics: | Finely granular | Finely granular | Complies |
| Rheology: | Free-flowing | Free-flowing | Complies |
| Taste: | Salty | Salty | Complies |
| Iodine test: | Negative | Negative | Complies |
| Moisture: | 2.2 - 7.0% | 3.70% | Complies |
| Solubility: | Complete | Complete | Complies |
| Minerals: | | | |
| Ca⁺⁺ | >25 mg/gram | 56.3 mg/gram | Complies |
| Mg⁺⁺ | >10 mg/gram | 14.3 mg/gram | Complies |
| Organic Acids: | | | |
| Citric: | Present | Present | Complies |
| Isocitric: | Present | Present | Complies |
| Lactic: | <25 mg/gram | 12 ppm | Complies |
| Malic: | >250 mg/gram | 410 mg/gram | Complies |
| Anthraquinones; | | | |
| Aloin A: | <0.05 ppm | 0.02 ppm | Complies |
| Aloin B: | <0.03 ppm | 0.01 ppm | Complies |
| Aloe-emodin | ppm | 0.005 ppm | Complies |
| Emodin: | <0.01 ppm | 0.001 ppm | Complies |
| Brix Value: | <1.0 | Negative | Complies |

**Table 4: SEC results for aloe preparation prior to polymannan extraction.**

| **Aloe** | | | | | |
|---|---|---|---|---|---|
| **#09116** | | | | | |
| **Retention Time** | **Refractive Index** | **Area** | **∑ Area** | **% Area** | **Molecular Size** |
| 0:30 | 0.0000 | 0.0000 | | | |
| 1:00 | 0.0000 | 0.0000 | | | |
| 1:30 | 0.0000 | 0.0000 | | | |
| 2:00 | 0.0000 | 0.0000 | | | |
| 2:30 | 0.0000 | 0.0000 | | | |
| 3:00 | 0.0000 | 0.0000 | | | |
| 3:30 | 0.0000 | 0.0000 | | | |
| 4:00 | 0.0000 | 0.0000 | | | |
| 4:30 | 0.0000 | 0.0000 | | | |
| 5:00 | 0.0000 | 0.0000 | | | |
| 5:30 | 0.0000 | 0.0000 | | | |
| 6:00 | 0.0000 | 0.0000 | | | |
| 6:30 | 0.0000 | 0.0000 | | | |
| 7:00 | 0.0000 | 0.0000 | | | |
| 7:30 | 0.0000 | 0.0000 | | | |
| 8:00 | 0.0000 | 0.0000 | | | |
| 8:30 | 0.0000 | 0.0000 | | | |
| 9:00 | 0.0000 | 0.0000 | | | |
| 9:30 | 0.0010 | 0.0014 | | | |
| 10:00 | 0.0020 | 0.0046 | | | |
| 10:30 | 0.0015 | 0.0058 | 0.012 | 2.44% | ***2 X 10⁶*** |
| 11:00 | 0.0010 | 0.0042 | | | |
| 11:30 | 0.0015 | 0.0039 | | | |
| 12:00 | 0.0015 | 0.0049 | | | |
| 12:30 | 0.0015 | 0.0049 | 0.018 | 3.69% | ***1 X 10⁶*** |
| 13:00 | 0.0015 | 0.0049 | | | |
| 13:30 | 0.0020 | 0.0056 | | | |
| 14:00 | 0.0020 | 0.0065 | | | |
| 14:30 | 0.0025 | 0.0072 | | | |
| 15:00 | 0.0030 | 0.0088 | | | |
| 15:30 | 0.0040 | 0.0111 | 0.044 | 9.09% | ***4.80 X 10⁵*** |
| 16:00 | 0.0050 | 0.0144 | | | |
| 16:30 | 0.0055 | 0.0169 | | | |
| 17:00 | 0.0060 | 0.0186 | | | |
| 17:30 | 0.0070 | 0.0209 | 0.071 | 14.59% | ***6.6 X 10⁴*** |
| 18:00 | 0.0080 | 0.0241 | | | |
| 18:30 | 0.0090 | 0.0274 | | | |
| 19:00 | 0.0110 | 0.0320 | | | |
| 19:30 | 0.0130 | 0.0385 | | | |
| 20:00 | 0.0155 | 0.0457 | | | |
| 20:30 | 0.0170 | 0.0524 | | | |
| 21:00 | 0.0240 | 0.0649 | | | |
| 21:30 | 0.0005 | 0.0457 | | | |
| 22:00 | 0.0004 | 0.0015 | | | |
| 22:30 | 0.0005 | 0.0014 | | | |
| 23:00 | 0.0004 | 0.0015 | | | |
| 23:30 | 0.0003 | 0.0012 | | | |
| 24:00 | 0.0000 | 0.0000 | 0.340 | 70.20% | |
| | 0.0000 | **0.4849** | | 100.00% | |

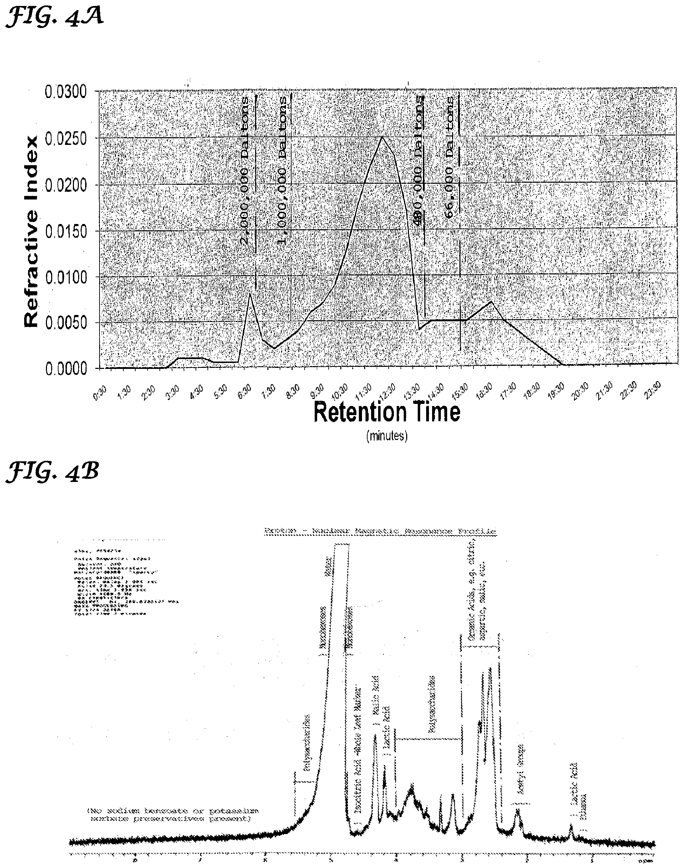

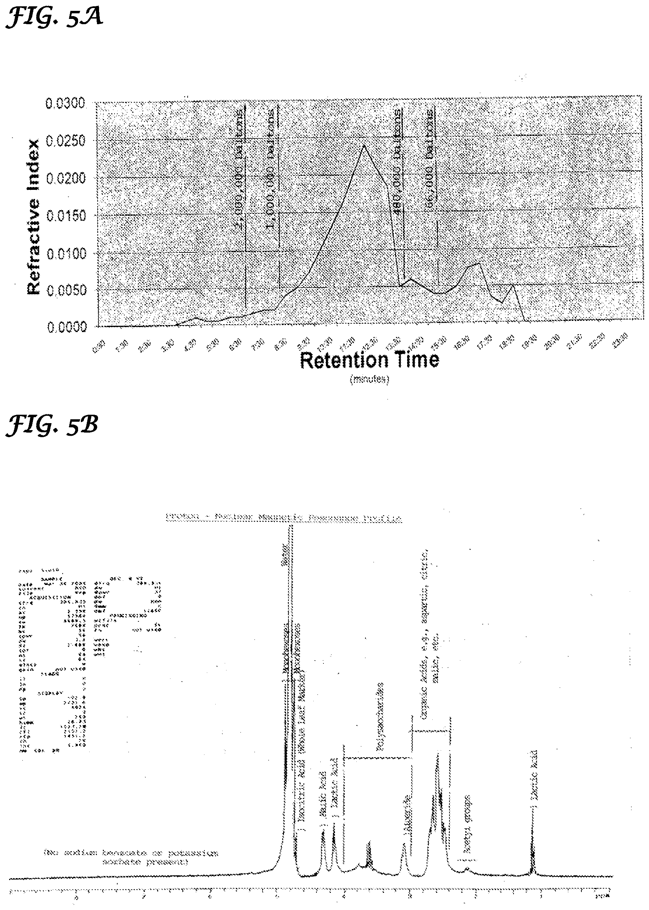

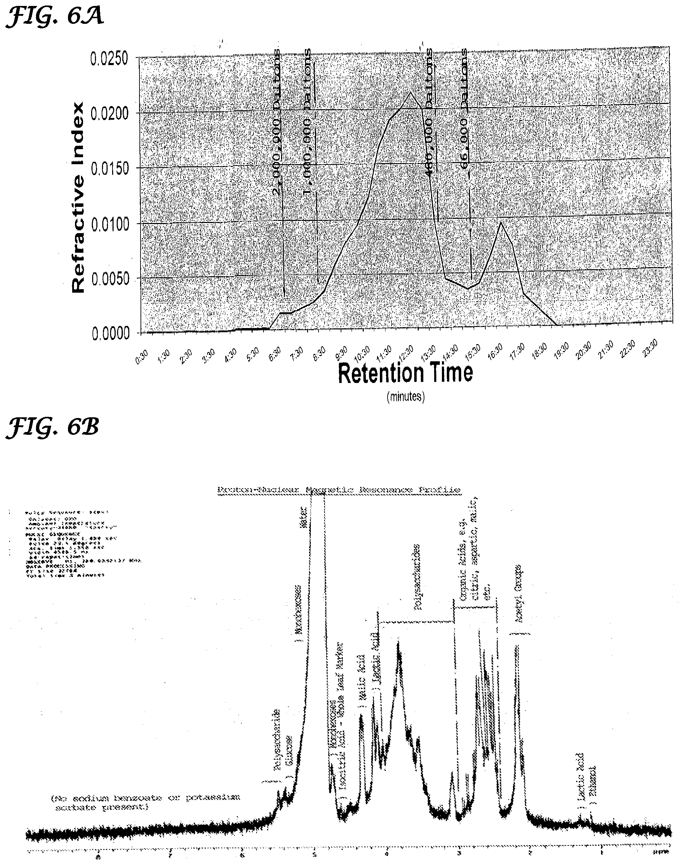

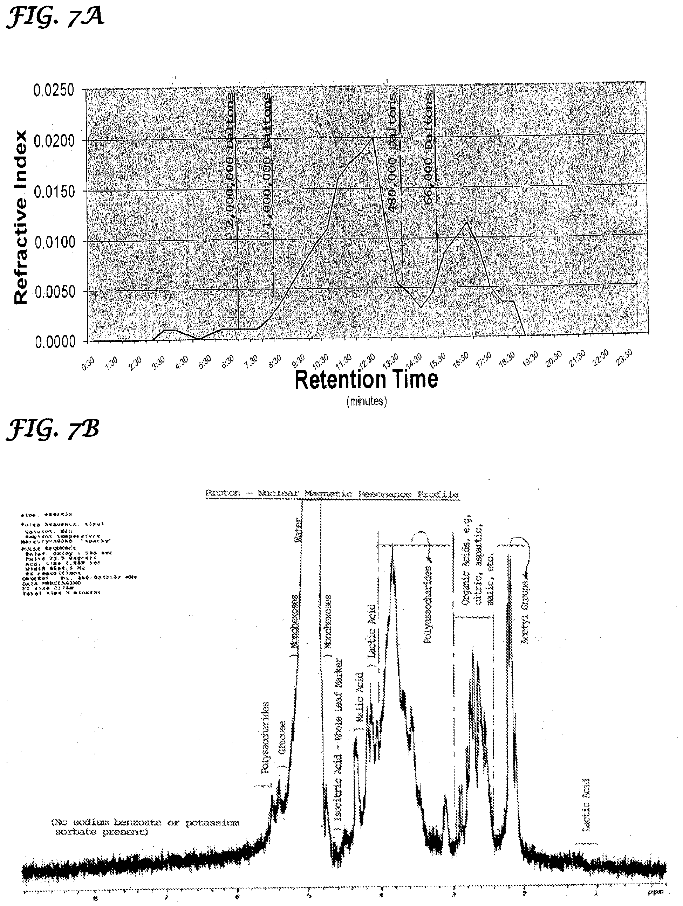

*Aloe precipitant evaluation study*: 25 ml samples of COATS concentrated aloe were pipetted into 200 ml beakers and 125 ml of various polysaccharide precipitant liquids were added to the beaker and thoroughly stirred. The precipitated polysaccharides were collected by filtration through tared dehydrated filter papers which, following filtration, were placed in the drying oven overnight. The next morning the dry weight of the precipitated polysaccharide was determined. The inventors studied four alcoholic precipitants were studied including, methanol, ethanol, isopropyl alcohol, and propanol. The powders were passed through a HPLC procedure which determined the various quantities of all of the molecular species which was recorded with determination of the amounts of polysaccharides in each of the polysaccharide molecular groups, including greater than 2,000,000 Daltons, greater than 1,000,000 Daltons, greater than 480,000 Daltons, greater than 66,000 Daltons, and the residual fraction containing the very small molecular species, e.g.. glucose having a M.W. of 180. (HPLC data shown in Tables 6-9). The HPLC chromatograms corresponding to the four precipitants methanol, ethanol, isopropyl alcohol, and propanol is shown in FIGS. 4A, 5A, 6A, and 7A, respectively. Proton Nuclear Magnetic Resonance Profiles of the precipitates were also obtained and are showin in FIGS. 4B, 5B, 6B, and 7B. The data collected is shown in Table 5.

**Table 5: Data from the aloe precipitant evaluation study.**

| Precipitant: | M.W. 2.0 x 10⁶ | M.W. 1.0 x 10⁶ | M.W. 4.8 x 10⁵ | M.W. 6.6 x 10⁴ | M.W. Residual |
|---|---|---|---|---|---|
| METHANOL | 6.36% | 3.89% | 68.56% | 6.92% | 14.27% |
| ETHANOL | 2.52% | 3.21% | 70.70% | 6.94% | 16.57% |
| ISOPROPYL ALCOHOL | 1.32% | 3.43% | 73.36% | 5.91% | 15.99% |
| PROPANOL | 2.74% | 2.55% | 63.85% | 6.92% | 23.94% |

**Table 6: HPLC data showing the amounts of polysaccharide in each polysaccharide molecular group in a methanol precipitated aloe polysaccharide concentrate.**

| **Aloe** | | | | | |
|---|---|---|---|---|---|
| **#09423-A** | | | | | |
| **Retention Time** | **Refractive Index** | **Area** | **∑ Area** | **% Area** | **Molecular Size** |
| 0:30 | 0.0000 | 0.0000 | | | |
| 1:00 | 0.0000 | 0.0000 | | | |
| 1:30 | 0.0000 | 0.0000 | | | |
| 2:00 | 0.0000 | 0.0000 | | | |
| 2:30 | 0.0000 | 0.0000 | | | |
| 3:00 | 0.0000 | 0.0000 | | | |
| 3:30 | 0.0010 | 0.0014 | | | |
| 4:00 | 0.0010 | 0.0033 | | | |
| 4:30 | 0.0010 | 0.0033 | | | |
| 5:00 | 0.0005 | 0.0026 | | | |
| 5:30 | 0.0005 | 0.0016 | | | |
| 6:00 | 0.0005 | 0.0016 | | | |
| 6:30 | 0.0080 | 0.0119 | | | |
| 7:00 | 0.0030 | 0.0191 | 0.045 | 6.36% | ***2 X 10⁶*** |
| 7:30 | 0.0020 | 0.0084 | | | |
| 8:00 | 0.0030 | 0.0079 | | | |
| 8:30 | 0.0040 | 0.0111 | 0.027 | 3.89% | ***1 X 10⁶*** |
| 9:00 | 0.0060 | 0.0158 | | | |
| 9:30 | 0.0070 | 0.0209 | | | |
| 10:00 | 0.0090 | 0.0255 | | | |
| 10:30 | 0.0130 | 0.0348 | | | |
| 11:00 | 0.0180 | 0.0491 | | | |
| 11:30 | 0.0220 | 0.0640 | | | |
| 12:00 | 0.0250 | 0.0756 | | | |
| 12:30 | 0.0230 | 0.0785 | | | |
| 13:00 | 0.0170 | 0.0665 | | | |
| 13:30 | 0.0040 | 00374 | | | |
| 14:00 | 0.0050 | 0.0144 | 0.482 | 68.56% | ***4.80 X 10⁵*** |
| 14:30 | 0.0050 | 0.0163 | | | |
| 15:00 | 0.0050 | 0.0163 | | | |
| 15:30 | 0.0050 | 0.0163 | 0.049 | 6.93% | ***6.6 X 10⁴*** |
| 16:00 | 0.0060 | 0.0176 | | | |
| 16:30 | 0.0070 | 0.0209 | | | |
| 17:00 | 0.0050 | 0.0200 | | | |
| 17:30 | 0.0040 | 0.0149 | | | |
| 18:00 | 0.0030 | 0.0116 | | | |
| 18:30 | 0.0020 | 0.0084 | | | |
| 19:00 | 0.0010 | 0.0051 | | | |
| 19:30 | 0.0000 | 0.0019 | | | |
| 20:00 | 0.0000 | 0.0000 | | | |
| 20:30 | 0.0000 | 0.0000 | | | |
| 21:00 | 0.0000 | 0.0000 | | | |
| 21:30 | 0.0000 | 0.0000 | | | |
| 22:00 | 0.0000 | 0.0000 | | | |
| 22:30 | 0.0000 | 0.0000 | | | |
| 23:00 | 0.0000 | 0.0000 | | | |
| 23:30 | 0.0000 | 0.0000 | | | |
| 24:00 | 0.0000 | 0.0000 | 0.100 | 14.27% | |
| | 0.0000 | **0.7036** | | 100.00% | |

**Table 7: HPLC data showing the amounts of polysaccharide in each polysaccharide molecular group in an ethanol precipitated aloe polysaccharide concentrate.**

| **Aloe** | | | | | |
|---|---|---|---|---|---|
| **#09423-B** | | | | | |
| **Retention Time** | **Refractive Index** | **Area** | **∑ Area** | **% Area** | **Molecular Size** |
| 0:30 | 0.0000 | 0.0000 | | | |
| 1:00 | 0.0000 | 0.0000 | | | |
| 1:30 | 0.0000 | 0.0000 | | | |
| 2:00 | 0.0000 | 0.0000 | | | |
| 2:30 | 0.0000 | 0.0000 | | | |
| 3:00 | 0.0000 | 0.0000 | | | |
| 3:30 | 0.0000 | 0.0000 | | | |
| 4:00 | 0.0005 | 0.0007 | | | |
| 4:30 | 0.0010 | 0.0023 | | | |
| 5:00 | 0.0005 | 0.0026 | | | |
| 5:30 | 0.0005 | 0.0016 | | | |
| 6:00 | 0.0010 | 0.0023 | | | |
| 6:30 | 0.0010 | 0.0033 | | | |
| 7:00 | 0.0015 | 0.0039 | 0.017 | 2.52% | ***2 X 10⁶*** |
| 7:30 | 0.0020 | 0.0056 | | | |
| 8:00 | 0.0020 | 0.0065 | | | |
| 8:30 | 0.0040 | 0.0093 | 0.021 | 3.21% | ***1 X 10⁶*** |
| 9:00 | 0.0050 | 0.0144 | | | |
| 9:30 | 0.0070 | 0.0190 | | | |
| 10:00 | 0.0100 | 0.0269 | | | |
| 10:30 | 0.0130 | 0.0366 | | | |
| 11:00 | 0.0160 | 0.0464 | | | |
| 11:30 | 0.0200 | 0.0575 | | | |
| 12:00 | 0.0240 | 0.0705 | | | |
| 12:30 | 0.0210 | 0.0739 | | | |
| 13:00 | 0.0185 | 0.0648 | | | |
| 13:30 | 0.0050 | 0.0416 | | | |
| 14:00 | 0.0060 | 0.0176 | 0.469 | 70.76% | ***4.80 X 10⁵*** |
| 14:30 | 0.0050 | 0.0181 | | | |
| 15:00 | 0.0040 | 0.0149 | | | |
| 15:30 | 0.0040 | 0.0130 | 0.046 | 6.94% | ***6.6 X 10⁴*** |
| 16:00 | 0.0050 | 0.0144 | | | |
| 16:30 | 0.0075 | 0.0197 | | | |
| 17:00 | 0.0080 | 0.0251 | | | |
| 17:30 | 0.0035 | 0.0198 | | | |
| 18:00 | 0.0025 | 0.0100 | | | |
| 18:30 | 0.0050 | 0.0116 | | | |
| 19:00 | 0.0000 | 0.0094 | | | |
| 19:30 | 0.0000 | 0.0000 | | | |
| 20:00 | 0.0000 | 0.0000 | | | |
| 20:30 | 0.0000 | 0.0000 | | | |
| 21:00 | 0.0000 | 0.0000 | | | |
| 21:30 | 0.0000 | 0.0000 | | | |
| 22:00 | 0.0000 | 0.0000 | | | |
| 22:30 | 0.0000 | 0.0000 | | | |
| 23:00 | 0.0000 | 0.0000 | | | |
| 23:30 | 0.0000 | 0.0000 | | | |
| 24:00 | 0.0000 | 0.0000 | 0.110 | 16.57% | |
| | 0.0000 | **0.6630** | | 100.00% | |

**Table 8: HPLC data showing the amounts of polysaccharide in each polysaccharide molecular group in an isopropyl alcohol precipitated aloe polysaccharide concentrate.**

| **Aloe** | | | | | |
|---|---|---|---|---|---|
| **#09423-C** | | | | | |
| **Retention Time** | **Refractive Index** | **Area** | **∑ Area** | **% Area** | **Molecular Size** |
| 0:30 | 0.0000 | 0.0000 | | | |
| 1:00 | 0.0000 | 0.0000 | | | |
| 1:30 | 0.0000 | 0.0000 | | | |
| 2:00 | 0.0000 | 0.0000 | | | |
| 2:30 | 0.0001 | 0.0001 | | | |
| 3:00 | 0.0000 | 0.0002 | | | |
| 3:30 | 0.0000 | 0.0000 | | | |
| 4:00 | 0.0000 | 0.0000 | | | |
| 4:30 | 0.0001 | 0.0001 | | | |
| 5:00 | 0.0001 | 0.0003 | | | |
| 5:30 | 0.0001 | 0.0003 | | | |
| 6:00 | 0.0001 | 0.0003 | | | |
| 6:30 | 0.0015 | 0.0023 | | | |
| 7:00 | 0.0015 | 0.0049 | 0.009 | 1.32% | ***2 X 10⁶*** |
| 7:30 | 0.0020 | 0.0056 | | | |
| 8:00 | 0.0025 | 0.0072 | | | |
| 8:30 | 0.0035 | 0.0095 | 0.022 | 3.43% | ***1 X 10⁶*** |
| 9:00 | 0.0060 | 0.0148 | | | |
| 9:30 | 0.0080 | 0.0223 | | | |
| 10:00 | 0.0095 | 0.0281 | | | |
| 10:30 | 0.0120 | 0.0343 | | | |
| 11:00 | 0.0165 | 0.0452 | | | |
| 11:30 | 0.0190 | 0.0571 | | | |
| 12:00 | 0.0200 | 0.0631 | | | |
| 12:30 | 0.0215 | 0.0671 | | | |
| 13:00 | 0.0200 | 0.0678 | | | |
| 13:30 | 0.0100 | 0.0513 | | | |
| 14:00 | 0.0045 | 0.0249 | 0.476 | 73.36% | ***4.80 X 10⁵*** |
| 14:30 | 0.0040 | 0.0139 | | | |
| 15:00 | 0.0035 | 0.0123 | | | |
| 15:30 | 0.0040 | 0.0121 | 0.038 | 5.91% | ***6.6 X 10⁴*** |
| 16:00 | 0.0065 | 0.0164 | | | |
| 16:30 | 0.0096 | 0.0254 | | | |
| 17:00 | 0.0075 | 0.0283 | | | |
| 17:30 | 0.0030 | 0.0182 | | | |
| 18:00 | 0.0020 | 0.0084 | | | |
| 18:30 | 0.0010 | 0.0051 | | | |
| 19:00 | 0.0000 | 0.0019 | | | |
| 19:30 | 0.0000 | 0.0000 | | | |
| 20:00 | 0.0000 | 0.0000 | | | |
| 20:30 | 0.0000 | 0.0000 | | | |
| 21:00 | 0.0000 | 0.0000 | | | |
| 21:30 | 0.0000 | 0.0000 | | | |
| 22:00 | 0.0000 | 0.0000 | | | |
| 22:30 | 0.0000 | 0.0000 | | | |
| 23:00 | 0.0000 | 0.0000 | | | |
| 23:30 | 0.0000 | 0.0000 | | | |
| 24:00 | 0.0000 | 0.0000 | 0.104 | 15.99% | |
| | 0.0000 | **0.6487** | | 100.00% | |

**Table 9: HPLC data showing the amounts of polysaccharide in each polysaccharide molecular group in a propanol precipitated aloe polysaccharide concentrate.**

| **Aloe** | | | | | |
|---|---|---|---|---|---|
| **#09423-C** | | | | | |
| **Retention Time** | **Refractive Index** | **Area** | **∑ Area** | **% Area** | **Molecular Size** |
| 0:30 | 0.0000 | 0.0000 | | | |
| 1:00 | 0.0000 | 0.0000 | | | |
| 1:30 | 0.0000 | 0.0000 | | | |
| 2:00 | 0.0000 | 0.0000 | | | |
| 2:30 | 0.0001 | 0.0001 | | | |
| 3:00 | 0.0000 | 0.0002 | | | |
| 3:30 | 0.0000 | 0.0000 | | | |
| 4:00 | 0.0000 | 0.0000 | | | |
| 4:30 | 0.0001 | 0.0001 | | | |
| 5:00 | 0.0001 | 0.0003 | | | |
| 5:30 | 0.0001 | 0.0003 | | | |
| 6:00 | 0.0001 | 0.0003 | | | |
| 6:30 | 0.0015 | 0.0023 | | | |
| 7:00 | 0.0015 | 0.0049 | 0.009 | 1.32% | ***2 X 10⁶*** |
| 7:30 | 0.0020 | 0.0056 | | | |
| 8:00 | 0.0025 | 0.0072 | | | |
| 8:30 | 0.0035 | 0.0095 | 0.022 | 3.43% . | ***1 X 10⁵*** |
| 9:00 | 0.0060 | 0.0148 | | | |
| 9:30 | 0.0080 | 0.0223 | | | |
| 10:00 | 0.0095 | 0.0281 | | | |
| 10:30 | 0.0120 | 0.0343 | | | |
| 11:00 | 0.0165 | 0.0452 | | | |
| 11:30 | 0.0190 | 0.0571 | | | |
| 12:00 | 0.0200 | 0.0631 | | | |
| 12:30 | 0.0215 | 0.0671 | | | |
| 13:00 | 0.0200 | 0.0678 | | | |
| 13:30 | 0.0100 | 0.0513 | | | |
| 14:00 | 0.0045 | 0.0249 | 0.476 | 73.36% | ***4.80 X 10⁵*** |
| 14:30 | 0.0040 | 0.0139 | | | |
| 15:00 | 0.0035 | 0.0123 | | | |
| 15:30 | 0.0040 | 0.0121 | 0.038 | 5.91% | ***6.6 X 10⁴*** |
| 16:00 | 0.0065 | 0.0164 | | | |
| 16:30 | 0.0096 | 0.0254 | | | |
| 17:00 | 0.0075 | 0.0283 | | | |
| 17:30 | 0.0030 | 0.0182 | | | |
| 18:00 | 0.0020 | 0.0084 | | | |
| 18:30 | 0.0010 | 0.0051 | | | |
| 19:00 | 0.0000 | 0.0019 | | | |
| 19:30 | 0.0000 | 0.0000 | | | |
| 20:00 | 0.0000 | 0.0000 | | | |
| 20:30 | 0.0000 | 0.0000 | | | |
| 21:00 | 0.0000 | 0.0000 | | | |
| 21:30 | 0.0000 | 0.0000 | | | |
| 22:00 | 0.0000 | 0.0000 | | | |
| 22:30 | 0.0000 | 0.0000 | | | |
| 23:00 | 0.0000 | 0.0000 | | | |
| 23:30 | 0.0000 | 0.0000 | | | |
| 24:00 | 0.0000 | 0.0000 | 0.104 | 15.99% | |
| | 0.0000 | **0.6487** | | 100.00% | |

The polymannan extract is prepared by precipitation. The freeze dried aloe powder is described above was weighed after correcting appropriately for the moisture content. For example, if the moisture content is 3.7% and we need 80 gms, the inventors weighed out 82. 96 gms (80 gms + (3.7% x 80) gms). The weighed aloe powder was dissolved completely in one gallon of deionized water (D.I) in a stainless steel precipitation vessel. 2.5 gallons of 95% ethanol was added and stirred to ensure complete mixing. The vessel was covered with a stainless steel lid and the mixture was allowed to settle overnight.

The following day a 2ml of clear supernatant was taken and 5 mL of 95% ethanol was added and the sample was centrifuged at 3000 rpm for 20 minutes. The sample was examined for precipitation, if no precipitate was observed then the precipitation was considered complete. If any significant degree of precipitation was observed then additional 95% ethanol was added to the precipitation vessel before proceeding. The clear supernatant fluid in the precipitation vessel was decanted by siphoning without disturbing the precipitate at the bottom of the vessel. The white precipitate at the bottom was separated by using a suction funnel (Whatman No. 42® quantitative ashless filter paper. The precipitated material was removed by scraping it into a 600 ml Virtis lyophilization flask, and by distributing the material over one side of the flask to form a thin layer with a large exposed surface area. The lyophilization flask was placed in a shell-freezer overnight. The next day the chilled lyophilization flask with its frozen contents, was placed on a lyophilizer operating at -90°C and at 1/3 atmosphere for 24 hours. The lyophizer was turned off and the lyophilized powder was placed into a small powder grinder until it reduced to an evenly ground fine powder. The ground powder was weighed and placed in plastic small containers and the containers were stored in a freezer.

*Preparation of an injectable solution of Polymannan extract*: The polymannan extract powder (PME) prepared as described above was weighed (1.5 gms) after correcting for moisture content and having an aloeride content of at least 2% as determined by size exclusion chromatography. To 125 ml of warm D.I water 1 ml of concentrated HCl was added and stirred followed by the slow addition of the PME powder with constant stirring. The stirring was continued till all the PME powder dissolved and the solution was clear and colorless an additional amount of concentrated HCl was added to obtain a pH of 1.6 to 1.7 (measured continuously using a pH meter). Additional D.I water was added to adjust the volume to 150 ml followed by a pH monitoring to ensure a pH of 1.6-1.7. The PME solution was then poured into a Corning® 150 ml filter system flask with a pore size of 0.45 µm. The flask system was placed in a refrigerator and the filtrate was transferred to a Corning® 150 ml filter system flask with a pore size of 0.22 µm and placed in a refrigerator overnight. Under sterile conditions the filter top of the filter system was removed and the bottle was sealed with a sterile cap. The bottles were them transferred to a compounding lab, and under a sterile hood 0.9% benzyl alcohol was added as a preservative (because the final product is for multi-dosage use), and the solution was placed in sterile 10 mL glass vials and sealed with a multidosage closure. The vials are labeled with a batch number, control number, manufacturing date, expiration date of 6 months along with the names of the physician and patient.

*PME immunomodulatory activity assessment*: The immune stimulatory activity is assessed using macrophages/monocytes of human origin obtained from the American Type Culture Collection (ATCC) in Maryland. The cell type was assessed for the secretion of TNFα. Under standard cell conditions a small amount of the final PME product was introduced in the culture. Samples were drawn at 6, 12, and 24 hours and assessed for TNFα levels. A specific quantity of TNFα was not used because of the variability in the different cell batches. In a clinical setting the immunomodulatory response is expected to vary due to changing hemotological factors like the total leukocyte count, differential macrophage/monocyte count, number of surface mannose receptors on the white cells, amount of mannose-binding carrier protein, etc.

The white blood cell profile varies with cells constantly entering and leaving the blood stream. The affinity of the cellular mannose receptors for the PME far exceeds that of the mannose binding protein. As new macrophages/monocytes enter the blood stream, the PME is transferred to the new cells from the circulating mannose-binding protein. PME binding to the macrophage/monocyte mannose-binding protein results in the release of an array of cytocommunicators. The cytocommunicators including TNF-α, IL-1β, INF-γ, IL-2, and IL-6 restore to normal the impaired surveillance function of the immune system which had failed in its neoplasm detection function in the cancer patient permitting the patient's immune system of identifying and removing the malignant cells.

Aloe polysaccharides in the polymannan extract having molecular weights of 1,000,000, 300,000, 100,000, 50,000 and 25,000 all showed caspase activity. This caspase 3, caspase 9, and cytochrome-C activity is key in the treatment of malignancies by the composition of the present invention, as caspase 3 is a mediator of tumor cell apoptosis. The immune modulatory activity of initiator (apical) caspase 3 and effector (executioner) caspase 3 as well as cytochrome-C have been demonstrated as being extant and are considered to be the mediator system of tumor cell apoptosis.

The inventors tested the composition described herein on 104 patients with different types of cancers. Leukemia and lymphomas were most responsive to the polymannan extract of the present invention (> 98%). Prostate, breast, and colon cancers were also responsive to the polymannan extract of the present invention. For the testing the polyamman extract was adminsiteres as an injection. 10 mg of the polymannan extract was reconstituted in sterile water for injection to give a final concentration of ∼ 10 mg/mL. This was injected 2 to 3 times a week. The serum samples from the patients were then taken at regular intervals and monitored for caspase 3 activity.

It is contemplated that any embodiment discussed in this specification can be implemented with respect to any method, kit, reagent, or composition of the invention, and vice versa. Furthermore, compositions of the invention can be used to achieve methods of the invention. It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

### REFERENCES

United States Patent No. 7,196,072: High Molecular Weight Polysaccharide Fraction From Aloe Vera with Immunostimulatory Activity.

United States Patent No. 6,083,508: Method of Processing Aloe Leaves.

United States Patent Application No. 2006/0084629: Immune System Activating Formula Composed of Selected Long Chain Polysaccharides From Natural Sources.

¹Pugh N., Ross S.A., ElSohly M.A., and Pasco, D.S. (2001). Characterization of Aloeride, a new high-molecular weight polysaccharide from Aloe vera with potent immunomodulatory activity. J Agr. Food Chem., 49, 1030-1034.

## Claims

1. A method for preparing a fine powder of a polymannan extract as end product comprising the steps of:
weighing a specified quantity of a freeze-dried aloe powder as source product, wherein the quantity is corrected for a moisture content;
dissolving the freeze-dried aloe powder in deionized water to form a solution;
adding a first volume of an alcoholic solvent to the solution to form a first mixture; wherein the alcoholic solvent to the deionized water ratio is at least 2.5:1;
allowing the first mixture to settle for at least 8 hours;
withdrawing a volume of a supernatant solution from the first mixture and adding a second volume of the alcoholic solvent to the volume of the supernatant solution to form a second mixture;
centrifuging the second mixture;
observing for a presence of a precipitate in the second mixture;
adding a third volume of the alcoholic solvent to the first mixture if any precipitate is observed in the second mixture;
decanting the supernatant of the first mixture by siphoning, wherein the decantation is done only if no precipitate is observed in the second mixture;
filtering the precipitate from the first mixture by using a filter paper and a suction funnel under vacuum;
recovering the powder of the polymannan extract from the suction funnel by scraping;
placing the powder of the polymannan extract in a capped lyophilization flask in a freezer for at least 8 hours;
lyophilizing the frozen powder of the polymannan extract in a lyophilizer; and
grinding the lyophilized powder of the polymannan extract in a grinder to a desired texture.

2. The method of claim 1, wherein the freeze-dried aloe powder source product is derived from an Aloe species selected from the group consisting of *Aloe vera, Aloe arborescens, Aloe aristata, Aloe dichotoma, Aloe nyeriensis, Aloe variegate, Aloe barbadensis, and Aloe wildii.*

3. The method of claim 1, wherein the alcoholic solvent is selected from the group consisting of methanol, ethanol, isopropyl alcohol, and propanol.

4. The method of claim 1, wherein the freeze-dried aloe powder source product comprises aloe polysaccharides having a molecular weight ranging from 2,000,000 to 10,000,000 Daltons.

5. The method of claim 1, wherein the freeze-dried aloe powder source product has at least 25%, 30%, 40%, 50%, 60%, 70%, 80%, 90%, and 95% aloe polysaccharides.

6. The method of claim 1, wherein the freeze dried aloe powder source product comprises optionally: at least 14% of aloe polysaccharides having a molecular weight of 66,000 Daltons; at least 9% of aloe polysaccharides having a molecular weight of 480,000 Daltons; at least 3.5% of aloe polysaccharides having a molecular weight of 1,000,000 Daltons; at least 2.4% of aloe polysaccharides having a molecular weight of 2,000,000 Daltons.

7. The method of claim 1, wherein the amount of aloe polysaccharides having a molecular weight of at least one of 2,000,000 Daltons in the freeze dried aloe powder source product ranges from 1.32%-6.36%; a molecular weight of 1,000,000 Daltons in the freeze dried aloe powder ranges from 2.55%-3.89%; a molecular weight of 480,000 Daltons in the freeze dried aloe powder ranges from 63.85%-73.36%; or wherein the fine powder of the polymannan extract is used on the preparation of a pharmaceutical composition.

8. The method of claim 1, wherein the freeze dried aloe powder source product may contain one or more residual small molecular weight species, selected from the group consisting of simple sugars, selected from the group consisting of glucose, mannose, arabinose, and galactose, organic acids, lactic acid, malic acids, citric acids, aspartic acid.

9. The method of claim 8, wherein the one or more residual small molecular weight species are present in amounts ranging from 14%-24%.

10. The method of claim 9, wherein the polymannan extract end product is used for the preparation of a pharmaceutical composition for use in the treatment of one or more malignancies selected from the group consisting of leukemias and lymphomas, prostate cancer, breast cancer, and colon cancer, and for the treatment of one or more immune disorders, or which causes a 75-80% increase in one or more natural killer (NK) cells.

11. A medicament for use in the treatment of one or more cancers which are selected from the group consisting of leukemias and lymphomas, prostate cancer, breast cancer, and colon cancer, comprising the steps of:
identifying an individual in need of treatment against the one or more cancers; and
injecting a sterile injectable Aloe vera polymannan extract obtainable by the method of claim 1 as end product in a formulation two to three times in a week in a dosage sufficient to treat the one or more cancers, wherein the sterile injectable Aloe vera polymannan extract formulation comprises a specified quantity of very fine Aloe vera polymannan extract dissolved in deionized water; and one or more pharmaceutical preservatives.

12. The medicament for use according to claim 11 further comprising the steps of:
withdrawing blood samples from the individual at one or more specified intervals; and
measuring a level of a caspase 3 protein in the blood and comparing the level obtained with the level prior to the injection, wherein an increased level in caspase 3 is directly related to an increased level of apoptosis of the one or more cancer cells.

13. The medicament for use according to claim 11, wherein the dosage of the sterile injectable polymannan extract formulation is dependent on a weight, an age, an ethnicity, and a gender of the individual.

14. The medicament for use according to claim 11, wherein the polymannan extract comprises aloe polysaccharides having a molecular weight ranging from 2,000,000 to 10,000,000 Daltons.

15. The medicament for use according to claim 11, wherein the polymannan extract has at least 25%, 30%, 40%, 50%, 60%, 70%, 80%, 90%, and 95% of aloe polysaccharides.

16. The medicament for use according to claim 11, wherein the polymannan extract causes a 75-80% increase in one or more natural killer (NK) cells.

## Patentansprüche

1. Verfahren zum Herstellen eines feinen Pulvers eines Polymannan-Extrakts als Endprodukt, welches die folgenden Schritte umfasst:
Wiegen einer spezifizierten Menge eines gefriergetrockneten Aloe-Pulvers als Ausgangsprodukt, wobei die Menge um den Feuchtigkeitsgehalt berichtigt ist;
Lösen des gefriergetrockneten Aloe-Pulvers in entionisiertem Wasser, um eine Lösung zu bilden;
Zugeben eines ersten Volumens eines alkoholischen Lösungsmittels zur Lösung, um ein erstes Gemisch zu bilden; wobei das Verhältnis des alkoholischen Lösungsmittels zum entionisierten Wasser mindestens 2,5:1 beträgt;
es dem ersten Gemisch erlauben, sich für mindestens 8 Stunden zu setzen;
Abziehen eines Volumens einer Flüssigkeitsüberstandlösung aus dem ersten Gemisch und Zugeben eines zweiten Volumens des alkoholischen Lösungsmittels zum Volumen der Flüssigkeitsüberstandlösung, um ein zweites Gemisch zu bilden;
Zentrifugieren des zweiten Gemisches;
Beobachten hinsichtlich der Anwesenheit eines Niederschlags im zweiten Gemisch;
Zugeben eines dritten Volumens des alkoholischen Lösungsmittels zum ersten Gemisch, falls ein Niederschlag im zweiten Gemisch beobachtet wird;
Dekantieren des Flüssigkeitsüberstands des ersten Gemisches durch Siphonieren, wobei das Dekantieren nur durchgeführt wird, wenn kein Niederschlag im zweiten Gemisch beobachtet wird;
Filtrieren des Niederschlags vom ersten Gemisch durch Verwenden eines Filterpapiers und eines Absaugtrichters unter Vakuum;
Rückgewinnen des Pulvers des Polymannan-Extrakts aus dem Absaugtrichter durch Kratzen;
Platzieren des Pulvers des Polymannan-Extrakts in einen abgedeckten Lyophilisierungskolben in einen Gefrierapparat für mindestens 8 Stunden;
Lyophilisieren des gefrorenen Pulvers des Polymannan-Extrakts in einem Gefriertrockenapparat; und Mahlen des lyophilisierten Pulvers des Polymannan-Extrakts in einer Mühle zu einer gewünschten Textur.

2. Verfahren nach Anspruch 1, wobei das gefriergetrocknete Aloe-Pulver-Ausgangsprodukt von einer Aloe-Art stammt, ausgewählt aus der Gruppe bestehend aus *Aloe vera, Aloe arborescens, Aloe aristata*, *Aloe dichotoma*, *Aloe nyeriensis*, *Aloe variegate, Aloe barbadensis* und *Aloe wildii.*

3. Verfahren nach Anspruch 1, wobei das alkoholische Lösungsmittel ausgewählt ist aus der Gruppe bestehend aus Methanol, Ethanol, Isopropylalkohol und Propanol.

4. Verfahren nach Anspruch 1, wobei das gefriergetrocknete Aloe-Pulver-Ausgangsprodukt Aloe-Polysaccharide mit einem Molekulargewicht im Bereich von 2.000.000 bis 10.000.000 Dalton umfasst.

5. Verfahren nach Anspruch 1, wobei das gefriergetrocknete Aloe-Pulver-Ausgangsprodukt mindestens 25%, 30%, 40%, 50%, 60%, 70%, 80%, 90% und 95% Aloe-Polysaccharide hat.

6. Verfahren nach Anspruch 1, wobei das gefriergetrocknete Aloe-Pulver-Ausgangsprodukt gegebenenfalls umfasst: mindestens 14% Aloe-Polysaccharide mit einem Molekulargewicht von 66.000 Dalton; mindestens 9% Aloe-Polysaccharide mit einem Molekulargewicht von 480.000 Dalton; mindestens 3,5% Aloe-Polysaccharide mit einem Molekulargewicht von 1.000.000 Dalton; mindestens 2,4% Aloe-Polysaccharide mit einem Molekulargewicht von 2.000.000 Dalton.

7. Verfahren nach Anspruch 1, wobei die Menge an Aloe-Polysacchariden mit einem Molekulargewicht von mindestens einem von 2.000.000 Dalton im gefriergetrockneten Aloe-Pulver-Ausgangsprodukt von 1,32%-6,36% reicht; einem Molekulargewicht von 1.000.000 Dalton im gefriergetrockneten Aloe-Pulver von 2,55%-3,89% reicht; einem Molekulargewicht von 480.000 Dalton im gefriergetrockneten Aloe-Pulver von 63,85%-73,36% reicht; oder worin das feine Pulver des Polymannan-Extrakts bei der Herstellung einer pharmazeutischen Zusammensetzung verwendet wird.

8. Verfahren nach Anspruch 1, wobei das gefriergetrocknete Aloe-Pulver-Ausgangsprodukt eine oder mehrere rückständige Arten mit kleinem Molekulargewicht enthalten kann, ausgewählt aus der Gruppe bestehend aus einfachen Zuckern, ausgewählt aus der Gruppe bestehend aus Glukose, Mannose, Arabinose und Galaktose, organischen Säuren, Milchsäure, Apfelsäure, Zitronensäure, Asparaginsäure.

9. Verfahren nach Anspruch 8, wobei die eine oder mehreren rückständigen Arten mit kleinem Molekulargewicht in Mengen im Bereich von 14%-24% vorhanden sind.

10. Verfahren nach Anspruch 9, wobei das Polymannan-Extrakt-Endprodukt für die Herstellung einer pharmazeutischen Zusammensetzung zur Verwendung in der Behandlung von einer oder mehreren Malignitäten verwendet wird, ausgewählt aus der Gruppe bestehend aus Leukämien und Lymphomen, Prostatakrebs, Brustkrebs und Darmkrebs, und für die Behandlung von einer oder mehreren Immunstörungen, oder welche einen 75-80% Anstieg bei einer oder mehreren natürlichen Killerzellen (NK-Zellen) verursacht.

11. Medikament zur Verwendung in der Behandlung von einem oder mehreren Krebsen, welche ausgewählt sind aus der Gruppe bestehend aus Leukämien und Lymphomen, Prostatakrebs, Brustkrebs und Darmkrebs, umfassend die Schritte:
Identifizieren eines Individuums, welches Behandlung gegen den einen oder mehrere Krebse benötigt; und
Injizieren eines sterilen injizierbaren Aloe-Vera-Polymannan-Extrakts, erhältlich durch das Verfahren nach Anspruch 1 als Endprodukt in einer Formulierung, zwei- oder dreimal in einer Woche in einer Dosierung, welche ausreichend ist, den einen oder die mehreren Krebse zu behandeln, wobei die sterile injizierbare Aloe-Vera-Polymannan-Extrakt-Formulierung eine spezifizierte Menge an sehr feinem Aloe-Vera-Polymannan-Extrakt, gelöst in entionisiertem Wasser; und einen oder mehrere pharmazeutische Konservierungsstoffe umfasst.

12. Medikament zur Verwendung gemäß Anspruch 11, ferner umfassend die Schritte:
Entnehmen von Blutproben aus dem Individuum bei einem oder mehreren spezifizierten Intervallen; und
Messen eines Grads eines Caspase-3-Proteins im Blut und Vergleichen des erhaltenen Grads mit dem Grad vor der Injektion, wobei ein erhöhter Grad an Caspase 3 in direkter Beziehung zu einem erhöhten Apoptosegrad der einen oder mehreren Krebszellen steht.

13. Medikament zur Verwendung gemäß Anspruch 11, wobei die Dosierung der sterilen injizierbaren Polymannan-Extrakt-Formulierung abhängig ist von Gewicht, Alter, Ethnizität und Geschlecht des Individuums.

14. Medikament zur Verwendung gemäß Anspruch 11, worin das Polymannan-Extrakt Aloe-Polysaccharide mit einem Molekulargewicht im Bereich von 2.000.000 bis 10.000.000 Dalton umfasst.

15. Medikament zur Verwendung gemäß Anspruch 11, worin das Polymannan-Extrakt mindestens 25%, 30%, 40%, 50%, 60%, 70%, 80%, 90% und 95% Aloe-Polysaccharide hat.

16. Medikament zur Verwendung gemäß Anspruch 11, wobei das Polymannan-Extrakt einen 75-80% Anstieg an einer oder mehreren natürlichen Killerzellen (NK-Zellen) verursacht.

## Revendications

1. Procédé pour préparer une poudre fine d'un extrait de polymannane en tant que produit final, comprenant les étapes consistant à :
peser une quantité spécifiée d'une poudre d'aloès lyophilisée en tant que produit source, laquelle quantité est corrigée pour la teneur en humidité ;
dissoudre la poudre d'aloès lyophilisée dans de l'eau désionisée pour former une solution ;
ajouter un premier volume d'un solvant alcoolique à la solution pour former un premier mélange ; le rapport du solvant alcoolique à l'eau désionisée étant d'au moins 2,5/1 ;
laisser le premier mélange sédimenter pendant au moins 8 heures ;
retirer un volume d'une solution de surnageant à partir du premier mélange et ajouter un deuxième volume du solvant alcoolique au volume de la solution de surnageant pour former un deuxième mélange ;
centrifuger le deuxième mélange ;
observer la présence d'un précipité dans le deuxième mélange ;
ajouter un troisième volume du solvant alcoolique au premier mélange si un quelconque précipité est observé dans le deuxième mélange ;
décanter le surnageant du premier mélange par siphonage, laquelle décantation est effectuée uniquement si aucun précipité n'est observé dans le deuxième mélange ;
filtrer le précipité issu du premier mélange par utilisation d'un filtre en papier et d'un entonnoir d'aspiration sous vide ;
récupérer la poudre de l'extrait de polymannane à partir de l'entonnoir d'aspiration par grattage ;
placer la poudre de l'extrait de polymannane dans un flacon de lyophilisation bouché dans un congélateur pendant au moins 8 heures ;
lyophiliser la poudre congelée de l'extrait de polymannane dans un lyophilisateur ; et
broyer la poudre lyophilisée de l'extrait de polymannane dans un broyeur jusqu'à une texture souhaitée.

2. Procédé selon la revendication 1, dans lequel le produit source de poudre d'aloès lyophilisée dérive d'une espèce d'aloès choisie dans le groupe constitué par *Aloe vera, Aloe arborescens, Aloe aristata*, *Aloe dichotoma*, *Aloe nyeriensis, Aloe variegate,*

3. Procédé selon la revendication 1, dans lequel le solvant alcoolique est choisi dans le groupe constitué par le méthanol, l'éthanol, l'alcool isopropylique, et le propanol.

4. Procédé selon la revendication 1, dans lequel le produit source de poudre d'aloès lyophilisée comprend des polysaccharides d'aloès ayant une masse moléculaire située dans la plage allant de 2 000 000 à 10 000 000 daltons.

5. Procédé selon la revendication 1, dans lequel le produit source de poudre d'aloès lyophilisée a au moins 25 %, 30 %, 40 %, 50 %, 60 %, 70 %, 80 %, 90 % et 95 % de polysaccharides d'aloès.

6. Procédé selon la revendication 1, dans lequel le produit source de poudre d'aloès lyophilisée comprend éventuellement : au moins 14 % de polysaccharides d'aloès ayant une masse moléculaire de 66 000 daltons ; au moins 9 % de polysaccharides d'aloès ayant une masse moléculaire de 480 000 daltons ; au moins 3,5 % de polysaccharides d'aloès ayant une masse moléculaire de 1 000 000 daltons ; au moins 2,4 % de polysaccharides d'aloès ayant une masse moléculaire de 2 000 000 daltons.

7. Procédé selon la revendication 1, dans lequel la quantité de polysaccharides d'aloès ayant une masse moléculaire d'au moins 2 000 000 daltons dans le produit source de poudre d'aloès lyophilisée est située dans la plage allant de 1,32 % à 6,36 % ; une masse moléculaire de 1 000 000 daltons dans le produit source de poudre d'aloès lyophilisée est située dans la plage allant de 2,55 % à 3,89 % ; une masse moléculaire de 480 000 daltons dans la poudre d'aloès lyophilisée est située dans la plage allant de 63,85 à 73,36 % ; ou dans lequel la poudre fine de l'extrait de polymannane est utilisée lors de la préparation d'une composition pharmaceutique.

8. Procédé selon la revendication 1, dans lequel le produit source de poudre d'aloès lyophilisée peut contenir une ou plusieurs espèces résiduelles de faible masse moléculaire, choisies dans le groupe constitué par les sucres simples, choisis dans le groupe constitué par le glucose, le mannose, l'arabinose et le galactose, les acides organiques, l'acide lactique, les acides maliques, les acides citriques, l'acide aspartique.

9. Procédé selon la revendication 8, dans lequel la ou les espèces résiduelles de faible masse moléculaire sont présentes en des quantités situées dans la plage allant de 14 % à 24 %.

10. Procédé selon la revendication 9, dans lequel le produit final d'extrait de polymannane est utilisé pour la préparation d'une composition pharmaceutique destinée à être utilisée dans le traitement d'une ou plusieurs malignités choisies dans le groupe constitué par les leucémies et les lymphomes, le cancer de la prostate, le cancer du sein et le cancer du côlon, et pour le traitement d'un ou plusieurs troubles immunitaires, ou qui provoque une augmentation de 75 à 80 % d'une ou plusieurs cellules tueuses naturelles (NK).

11. Médicament pour une utilisation dans le traitement d'un ou plusieurs cancers qui sont choisis dans le groupe constitué par les leucémies et les lymphomes, le cancer de la prostate, le cancer du sein et le cancer du côlon, comprenant les étapes consistant à :
identifier un individu ayant besoin d'être traité contre le ou les cancers ; et
injecter un extrait de polymannane d'Aloe vera injectable stérile pouvant être obtenu par le procédé de la revendication 1 en tant que produit final dans une formulation deux à trois fois par semaine à une dose suffisante pour traiter le ou les cancers,
la formulation d'extrait de polymannane d'Aloe vera injectable stérile comprenant une quantité spécifiée d'extrait de polymannane d'Aloe vera très fin dissous dans de l'eau désionisée ; et un ou plusieurs conservateurs pharmaceutiques.

12. Médicament pour une utilisation selon la revendication 11, comprenant en outre les étapes consistant à :
prélever des échantillons de sang de l'individu à un ou plusieurs intervalles spécifiés ; et
mesurer le niveau de protéine caspase 3 dans le sang et comparer le niveau obtenu avec le niveau avant l'injection,
un niveau accru de caspase 3 étant directement lié à un niveau accru d'apoptose de la ou des cellules cancéreuses.

13. Médicament pour une utilisation selon la revendication 11, dans lequel la dose de la formulation d'extrait de polymannane injectable stérile dépend du poids, de l'âge, de l'ethnicité et du sexe de l'individu.

14. Médicament pour une utilisation selon la revendication 11, dans lequel l'extrait de polymannane comprend des polysaccharides d'aloès ayant une masse moléculaire située dans la plage allant de 2 000 000 à 10 000 000 daltons.

15. Médicament pour une utilisation selon la revendication 11, dans lequel l'extrait de polymannane a au moins 25 %, 30 %, 40 %, 50 %, 60 %, 70 %, 80 %, 90 % et 95 % de polysaccharides d'aloès.

16. Médicament pour une utilisation selon la revendication 11, dans lequel l'extrait de polymannane provoque une augmentation de 75 à 80 % d'une ou plusieurs cellules tueuses naturelles (NK).
